(19) Europäisches Patentamt
European Patent Office
Office européen des brevets

(11) **EP 2 744 355 B1**

(12) **EUROPEAN PATENT SPECIFICATION**

(45) Date of publication and mention
of the grant of the patent:
**21.09.2016 Bulletin 2016/38**

(21) Application number: **12732612.2**

(22) Date of filing: **26.06.2012**

(51) Int Cl.:
*A23L 33/10* *(2016.01)*          *A23L 33/17* *(2016.01)*
*A61K 31/56* *(2006.01)*

(86) International application number:
**PCT/EP2012/062320**

(87) International publication number:
**WO 2013/023826 (21.02.2013 Gazette 2013/08)**

(54) **EDIBLE COMPOSITION COMPRISING STEROL**

ESSBARE ZUSAMMENSETZUNG MIT STEROL

COMPOSITION COMESTIBLE COMPRENANT DU STÉROL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.08.2011 EP 11177563**

(43) Date of publication of application:
**25.06.2014 Bulletin 2014/26**

(73) Proprietors:
• **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**
• **Unilever PLC**
**London, Greater London EC4Y 0DY (GB)**
Designated Contracting States:
**CY GB IE MT**

(72) Inventors:
• **DUCHATEAU, Gustaaf, Servaas, Marie, Joseph, Emile**
**NL-3133 AT Vlaardingen (NL)**
• **SMIT-KINGMA, Irene, Erica**
**NL-3133 AT Vlaardingen (NL)**

(74) Representative: **Wurfbain, Gilles L.**
**Unilever N.V.**
**Unilever Patent Group**
**Olivier van Noortlaan 120**
**3133 AT Vlaardingen (NL)**

(56) References cited:
EP-A1- 1 142 494          EP-A1- 1 839 501
WO-A1-03/055324          WO-A1-2008/125380
WO-A1-2011/144405

**Description**

**Field of the invention**

The invention relates to edible compositions comprising sterol for use in a treatment to lower blood cholesterol level.

**Background of the invention**

Plant sterols are well known cholesterol lowering agents. The benefit of these ingredients to reduce the risk to cardiovascular diseases has been established for many years. Where these active ingredients were initially available in the form of capsules and other pharmaceutical preparations only, over the years they have also become available in food products. The incorporation of these active ingredients in food products that are consumed daily enables the easy and reliable intake of these ingredients for many people.

Plant sterols as such are difficult to formulate into food products due to their poor solubility in oil and immiscibility with water which may result in food products having poor organoleptic properties, e.g. a sandy mouth feel. This made the choice of food products suitable for incorporation of plant sterols very limited. To overcome this drawback plant sterols have been modified to improve their solubility in the fat phase of food products. The most common modification of plant sterols is to their corresponding fatty acid esters. Commercial products such as Becel pro-activ[tm] and Senecol[tm] comprise sterol fatty acid esters.

It is desired that food products comprising plant sterols for use in a treatment to lower blood cholesterol are able to achieve a reduction in blood cholesterol level, preferably a large reduction in blood cholesterol level. This may reduce the required amount of plant sterols in said food products and as such may mitigate formulation problems of plant sterols in foods. Reducing the required amount of plant sterols will also reduce the cost of preparing such foods as plant sterols are relatively expensive food components. It will be appreciated that an edible food composition which is more effective in a treatment to reduce blood cholesterol level is also desirable as it may more effectively improve and/or maintain a healthy physiology.

Mattson et. al. (1982, Am J Clin Nutr) studied the bioefficacy of sterols added to a meal comprising scrambled eggs and 500 mg cholesterol. Addition of $\beta$-sitosterol (non-esterified sterols) resulted in a 42% decrease in cholesterol absorption and the addition of $\beta$-sitosteryl oleate in a 33% reduction.

Pouteau et. al. (2003, Eur J Nutr) studied the cholesterol inhibiting properties of verified properly solubilised, non-esterified plant sterols in partly vegetable oil containing milks. Sixteen hypercholeterolemic dult men consumed milk containing sterols (1.8 g of non-esterified pure plant sterols per day). The plant sterols contained very few crystals with a size > 11 micrometer. Cholesterol absorption was reduced to 41.1 % (from 70.1 %).

Vanstone et. al. (2002, Am J Clin Nutr) examined the effect of supplementation with unesterified plant sterols and stanols on plasma lipid and phytosterol concentrations and cholesterol absorption, synthesis and turnover. Fifteen hypercholesterolemic subjects consumed butter enriched with unesterified sterols and stanols at a dosage of 1.8 g per day. Cholesterol absorption was reduced by 56 % by a diet containing plant sterols, 34.4 % by a diet containing plant stanols and 48.9% by a diet containing 50:50 mixture of plant sterols and stanols.

Richelle et. al. (2004, Am J Clin Nutr) specifically compared the effects of plant stanol esters and plant free sterols on cholesterol absorption. A low-fat milk-based beverage comprising 2.2 g plant sterol equivalents was consumed per day. Both milks enriched with plant sterols induced a 60% decrease in cholesterol absorption.

Shin et. al. (2004, Ann Nutr & Met) evaluated effects of micellar phytosterols on cholesterol absorption. 500kcal test meals consisting of 70 g white bread, 2.7 g soybean oil comprising cholesterol, 200 ml milk, 20 g cheese and 10 g ham was consumed together with 200, 300 or 500 mg phytosterols. Cholesterol absorption was reduced up to 32% from normal.

Sudhop et. al. (2003, Drug Res.) evaluated the effect of unesterified sitostanol, sitostanol acetate and sitostanol oleate on cholesterol absorption, whereof 1.5 gram per day was administered via a margarine. Cholesterol absorption was estimated based on cholesterol content of fecal samples. A maximum reduction in the cholesterol absorption of 76% was observed, in comparison with a placebo, when unesterified sitostanol was administered.

It is an object of the present invention to provide edible compositions which are suitable for use in a treatment to lower blood cholesterol level. It is a further object of the invention to provide edible compositions for use in a treatment to lower blood cholesterol level with improved effectiveness.

**Summary of the invention**

It was found that one or more of the objectives mentioned above is attained by edible compositions comprising an aqueous phase, a fat phase, plant sterol particles and a water soluble biopolymer based emulsifier, wherein the food product is prepared by a process wherein fat powder comprising structuring fat is used.

Surprisingly it was found that the combination of certain water soluble emulsifiers, plant sterol particles of a certain size and a food product made by a process wherein fat powder comprising structuring fat is used, improves the effectiveness of a treatment to lower the blood cholesterol level.

Accordingly, the invention relates to an edible composition comprising an aqueous phase, a fat phase and plant sterol particles for use in a treatment to lower blood cholesterol, wherein said edible composition comprises from 0.1 to 20 wt. % of plant sterol particles whereof at least 60 wt. %, based on the total weight of said plant sterol particles, is comprised by said aqueous phase, and wherein at least 70 vol. % of said plant sterol particles is smaller than 10 micrometer, and wherein said edible composition comprises a water soluble biopolymer based emulsifier with a molecular weight of at least 500, and wherein said edible composition is made in a process using fat powder comprising structuring fat, and wherein said treatment comprises the step of consuming said edible composition in an amount comprising at least 0.3 g of said plant sterols per day.

The invention also relates to a process for the preparation of said edible composition.

**Detailed description of the invention**

Wt. % is calculated on weight of total product unless otherwise specified. For the purpose of the invention ambient temperature is defined as a temperature between 15 and 25 °C. The terms 'oil' and 'fat' are used interchangeably unless specified otherwise. The terms 'plant sterol', 'phytosterol' and 'sterol' are used interchangeably unless specified otherwise. The terms 'blood cholesterol level' and blood cholesterol concentration' are used interchangeably unless specified otherwise.

Plant sterol particles

Plant sterols can be classified in three groups, 4-desmethylsterols, 4-monomethylsterols and 4,4'-dimethylsterols. In oils they mainly exist as free sterols and sterol esters of fatty acids although sterol glucosides and acylated sterol glucosides are also present. There are three major phytosterols namely beta-sitosterol, stigmasterol and campesterol. Schematic drawings of the components meant are as given in "Influence of Processing on Sterols of Edible Vegetable Oils", S.P. Kochhar; Prog. Lipid Res. 22: pp. 161-188.

The respective 5 alpha- saturated derivatives such as sitostanol, campestanol and ergostanol and their derivatives are also encompassed in the term plant sterol.

Preferably the plant sterol is selected from the group comprising β-sitosterol, β-sitostanol, campesterol, campestanol, stigmasterol, brassicasterol, brassicastanol or a mixture thereof. Suitable sources of plant sterols are for example derived from soy bean oil, tall oil, rapeseed oil or combinations of these oils.

In the context of this invention the term plant sterol refers to the free plant sterol, i.e. the non-esterified plant sterol, unless specified otherwise.

The plant sterol particles need to have a certain particle size distribution. In the context of this invention the plant sterol particle size distribution is expressed as volume % (vol. %) calculated on total volume of the plant particles below a set particle size in micrometer. At least 70 vol. % of the plant sterol particles is smaller than 10 micrometer as this size distribution in combination with a water soluble emulsifier provides an edible composition for use in a treatment to lower blood cholesterol level with improved effectiveness. Without whishing to be bound by theory, it is believed that plant sterol particles smaller then 10 micrometer have improved kinetics of cholesterol binding in the digestive tract. Preferably at least 75 vol. %, more preferably at least 80 vol. %, even more preferably at least 85 vol. %, still more preferably at least 90 vol. % and even still more preferably at least 95 vol. % of the plant sterol particles are smaller than 10 micrometer.

At least 60 wt. % of the plant sterol particles, based of the total weight of said plant sterol particles, need to be comprised by the aqueous phase of the edible composition. Preferably at least 70 wt. %, more preferably at least 85 wt. % and most preferably at least 90 wt. % of said plant sterol particles, based of the total weight of said sterol particles, is comprised by said aqueous phase. It is believed that the presence of plant sterol particles in the aqueous phase improves the effectiveness of the edible composition to lower blood cholesterol level. Without wishing to be bound by theory, it is believed that the presence of the plant sterols in the aqueous phase improves the kinetics of cholesterol binding in the digestive tract.

Suitable methods for reducing the particle size of plant sterols to obtain plant sterols particles with a size distribution according to the invention are well known. These methods can roughly be divided in two approaches: top-down and bottom-up. In the top-down approach large particles are broken down to smaller ones using mechanical energy. Examples of such processes are melting and emulsification (e.g. as disclosed in WO0228204), and melt spraying and milling (e.g. as disclosed in WO9858554). In the bottom-up approach molecules, monomers or ions are condensed into a liquid or a solid phase using a physical or a chemical process. Examples of such processes are anti-solvent precipitation (e.g. as disclosed in US 2006/0035871 A1) and Rapid Expansion of a Supercritical Solution (RESS) as (disclosed in WO0021490). Powders can be obtained from the dispersions after drying using spray drying, freeze drying or any other suitable drying method.

WO2011144405 is an Art. 54(3) EPC document, which discloses an edible fat continuous spread being a water in oil emulsion comprising a water phase and a fat phase, wherein the fat phase comprises liquid oil and a structuring fat, said spread comprising a first emulsifier and a second emulsifier, 5 to 85 wt% fat and 0.1 to 20 wt% plant sterol particles characterized in that wherein the first emulsifier is a water soluble biopolymer based emulsifier with a molecular weight of at least 500, the second emulsifier is an oil soluble emulsifier and at least 70 vol% of the plant sterol particles is smaller than 10 micrometer.

EP1142494 discloses a plant sterol-emulsifier dispersion comprising a mixture of 1 to 80 percent of a plant sterol-emulsifier blend and 20 to 99 percent of a solvent for use in reducing serum cholesterol level. EP1839501 discloses compositions comprising phytosterols or phytostanols and emulsifiers for use in reducing blood cholesterol level. WO2008125380 discloses an edible fat continuous spread being a water in oil emulsion comprising a water phase and a fat phase, wherein the fat phase comprises liquid oil and a structuring fat, said spread comprising 5 to 85 wt% fat and from 0.1 to 20 wt% plant sterol wherein the plant sterol is present in the form of elongated crystals.

The edible composition according to the invention comprises from 0.1 to 20 wt. % plant sterol particles. It will be appreciated that the amount of plant sterol particles can suitable be adjusted to fit the dietary needs of the intended consumer of such an edible composition. Preferably the amount of plant sterol particles is from 2 to 15 wt. %, more preferably from 4 to 10 wt. % and even more preferably from 6 to 8 wt. %.

Water soluble biopolymer based emulsifier

Emulsifiers are well known food ingredients and can be divided in two classes, being water soluble emulsifiers and oil soluble emulsifiers. The water soluble emulsifier must be a biopolymer based emulsifier with a molecular weight of at least 500 and preferably the water soluble emulsifier is selected from the group consisting of proteins, glycoproteins, surface active polysaccharides and combinations thereof.

Suitable proteins are plant derived proteins like for example soy protein and animal derived proteins like for example gelatin and milk derived protein. Milk protein is also known as dairy protein. The molecular weight of said proteins can be determined with any of the generally known techniques for such purpose. Preferably the protein is selected from the group consisting of plant derived protein, animal derived protein and combinations thereof.

Preferably the water soluble emulsifier is a milk derived protein, and more preferably the protein comprises whey protein or casein protein, as for example can be found in full milk powder, skimmed milk powder, butter milk powder and sweet whey powder. Standard milk powder comprises about 35 wt. % of milk protein. This means that to include for example 0.5 wt. % water soluble emulsifier in a edible composition about 1.4 wt. % milk powder has to be added, of course depending on the actual amount of protein present in the milk powder used.

It is possible to use combinations of more than one water soluble emulsifier like for example protein comprising emulsifiers and/or specific milk proteins. This may be desired e.g. to get an optimal flavour and/or nutritional profile. Therefore, the water soluble emulsifier is preferably selected from the group consisting of full milk powder, skim milk powder, butter milk powder, sweet whey powder, whey protein, casein protein and combinations thereof.

Preferably, the edible composition according to the invention comprises an amount of water soluble emulsifier from 0.01 to 5 wt. %, more preferably from 0.1 to 2 wt. % and even more preferably from 0.1 to 0.5 wt. %.

Preferably, the edible composition according to the invention comprises a weight ratio of water soluble emulsifier to plant sterol particles from 10:1 to 1:80, more preferably 8:1 to 1:40, even more preferably 6:1 to 1:20, still more preferably 4:1 to 1:15 and even still more preferably 2:1 to 1:10.

Fat powder

The edible composition is made in a process wherein fat powder comprising structuring fat (i.e. hardstock) is used. An edible composition made using said fat powder improves the effectiveness of a treatment to lower blood cholesterol, when used in said treatment. Preferably, the edible composition is made in a process wherein the amount of fat powder used is from 1 to 20 wt. %, more preferably from 2 to 15 wt. %, even more preferably from 4 to 12 wt. %, still more preferably from 6 to 8 wt. % and even still more preferably from 3 to 5 wt. %, based on the total weight of the ingredients.

Furthermore, the presence of structuring fat may help to stabilize the edible composition. The crystallization and melting properties of the structuring fat are important as they influence the stability of the emulsion, e.g. syneresis and plasticity, as well as the organoleptic properties, e.g. oral melting behaviour and flavour release.

It will be appreciated that the amount of structuring fat necessary for imparting structure to an edible composition depends on the desired structure. For example, for a stable spread a certain amount of structuring fat is necessary. If the amount of structuring fat is too low, a stable emulsion may not be obtained and the resulting emulsion may not comprise the typical plasticity of a spread.

The fat powder comprises structuring fat and preferably comprises at least 80 wt. % of structuring fat, more preferably at least 85 wt. %, even more preferably at least 90 wt. %, still more preferably at least 95 wt. % and most preferably at least 98 wt. %. Most preferably the edible fat powder essentially consists of structuring fat.

The structuring fat may be a single fat or a mixture of different fats. The structuring fat may be of vegetable, animal or marine origin. Preferably at least 50 wt. % of the structuring fat (based on total amount of structuring fat) is of vegetable origin, more preferably at least 60 wt. %, even more preferably at least 70 wt. %, still more preferably at least 80 wt. %, even still more preferably at least 90 wt. % and even still more further preferably at least 95 wt. %. Most preferably the structuring fat essentially consists of structuring fat of vegetable origin.

The fat powder may comprise any suitable oil or fat. Preferred oils and fats are those known for the production of margarine and margarine derivatives such as low fat spreads. The oil and fat are for example selected from the group comprising sunflower oil, rapeseed oil, palm oil, coconut oil, soy bean oil, palm kernel oil, butter fat or a combination thereof. Preferably the liquid oil is selected from the group consisting of sunflower oil, rapeseed oil, soybean oil, linseed oil, maize oil and combinations thereof. Preferably the structuring fat is selected from the group consisting of palm oil, palm kernel oil, coconut oil and combinations thereof.

The structuring fat as present in the edible fat powder preferably has a solid fat content N10 from 50 to 100, N20 from 26 to 95 and N35 from 5 to 60.

Preferably, the structuring fat is made by a method such as Super Critical Melt Micronisation (ScMM), also known as particles from gas saturated solutions (PGSS). This is a commonly known method and is for example described in J. of Supercritical Fluids 43 (2007) 181-190 and EP1651338.

It is important that the fat powder is not subjected to temperatures at which the structuring fat melts as this may severely reduces the effectiveness of the use of the edible composition in a treatment to reduce blood cholesterol levels. This temperature depends on the structuring fat as used and can routinely be determined for example based on the solid fat content profile (i.e. N-lines) of the structuring fat. Preferably the fat powder, after production, has not been subjected to temperatures above 25 degrees Celsius, more preferably 15, even more preferably 10 and most preferably 5.

Treatment to lower blood cholesterol level

Cholesterol is used to produce hormones and cell membranes and is transported in the blood plasma of all mammals. It is an essential structural component of mammalian cell membranes and is required to establish proper membrane permeability and fluidity. In addition, cholesterol is an important component for the manufacture of bile acids, steroid hormones, and vitamin D. The concentration of cholesterol in the blood (a.k.a. blood cholesterol level) may vary between individuals. This variation may be caused by a number of things, such as differences in lifestyle, in particular dietary habits, but also differences in for example genetic background. What is to be considered as a healthy level of blood cholesterol, therefore, may vary from individual to individual and may be determined with the assistance of a trained physician. Any blood cholesterol level above such a healthy level is considered to be an elevated blood cholesterol level. Elevated (i.e. high) levels of blood cholesterol may affect health and may be associated with progression of cardio-vascular disease like for example atherosclerosis. Prolonged elevation of blood cholesterol may increase the risk of health related diseases and, for example, contribute to formation of atheromatous plaques in the arteries. This may lead to progressive stenosis (narrowing) or even complete occlusion (blockage) of the involved arteries.

The edible composition according to the invention is used by an individual in a treatment to lower blood cholesterol level or maintain a healthy blood cholesterol level. It will be appreciated that the edible composition according to the invention may be used by an individual for the prophylactic or curative treatment of elevated blood cholesterol level. Preferably, said treatment is used by a subject, wherein said subject preferably is a human, more preferably a human with an elevated blood cholesterol level, and most preferably a human with hypercholesterolemia.

Hypercholesterolemia is the presence of high levels of cholesterol in the blood, which may lead to specific physical findings such as xanthelasma palpebrarum (yellowish patches underneath the skin around the eyelids, arcus senilis (white or gray discoloration of the peripheral cornea), and xanthomata (deposition of yellowish cholesterol-rich material) of the tendons, especially of the fingers.

For ease of use and ease of adherence to a treatment to reduce blood cholesterol level, the edible composition is preferably in a food format which may suitably be consumed as such (e.g. a mini-drink) or in combination with other foods (e.g. a spread). Preferably, the edible composition is in the form of a spread, dressing, mayonnaise, shortening, filling, topping, cream, soup, beverage, sauce, mini-drink or snack. Preferably, the edible composition is a water-in-oil emulsion, more preferably a water-in-oil emulsion spread.

A reduction of blood cholesterol level results from a reduced uptake of cholesterol derived from foods and/or by a reduced uptake of cholesterol derived from bile. Bile (a.k.a. gall) is produced by the liver of most vertebrates and aids the process of digestion of lipids in the small intestine. In many species bile is stored in the gallbladder and upon eating may be discharged into the gastro intestinal tract, typically into the duodenum. The secretion of bile in the gut is stimulated by consumption of certain amounts of fat, proteins or carbohydrate, whereof fats are thought to most effectively stimulate bile release. Such an amount of fats and/or proteins and/or carbohydrates can typically be found in a meal. A meal is defined as an instance of eating, specifically one that takes place at a certain time and typically includes prepared food. Meals typically occur at homes, restaurants, and cafeterias, but may occur anywhere. Regular meals occur on a daily basis, typically several times a day. Typical examples of meals are the well known breakfast, lunch and dinner. The consumption of high amounts of fats, however, is undesired as this may itself lead to health problems, such as increase the risk of obesity. The edible composition according to the invention, for use in a treatment to lower blood cholesterol, is preferably consumed as a meal, or as part of a meal, more preferably wherein said meal comprises from 2 to 50 grams of fat, even more preferably from 3 to 10 grams of fat and most preferably from 4 to 6 grams of fat.

For an effective treatment to lower blood cholesterol level, the edible composition according to the invention is to be consumed in an amount comprising at least 0.3 g of said plant sterol particles per day. Said amount of edible product comprising 0.3 g of said sterols may be consumed in the course of a single eating or in the course of more then one eating. Said amount of edible product may be suitably be comprised by one or more types of food formats. An example of a treatment, according to the invention wherein food compositions according to the invention are consumed at different times during the day in different food formats is wherein, 10 gram of a spread comprising 5 wt.% sterols (i.e. 0.5 gram of sterol) is consumed at breakfast, a mini-drink comprising 1 gram of sterol is consumed as a snack, and 300 ml of a soup comprising 0.5 wt. % sterols (i.e. 1.5 gram of sterol) is consumed at dinner, resulting in a daily consumption of 3 gram of sterol. A second example of a treatment, according to the invention, wherein a single food composition according to the invention is consumed at different times during the day is wherein 10 gram of a spread comprising 10 wt. % sterols (i.e. 1 gram of sterol) is consumed at breakfast, 10 gram of said spread during lunch, and 10 gram of said spread during dinner, resulting in a daily consumption of 3 gram of sterol. A third example of a treatment, according to the invention, wherein a single food composition according to the invention is consumed at a single instance of eating is wherein 225 ml of a yoghurt-type dairy product comprising 1 wt. % of sterols is consumed at breakfast, resulting in a daily consumption of 2.25 gram of sterol. A fourth example of a treatment, according to the invention, wherein different food compositions according to the invention are consumed at a single instance of eating is wherein 200 ml of a yoghurt-type dairy product comprising 1 wt. % of sterols and a mini-drink comprising a total of 2 gram of sterol is consumed at breakfast, resulting in a daily consumption of 4 gram of sterol. It will be appreciated that the effectiveness of a treatment to lower blood cholesterol level may vary between individuals. To promote effectiveness over a wide range of individuals, preferably the edible composition is consumed in an amount of at least 0.5 gram, more preferably at least 1 gram, even more preferably at least 2 gram and most preferably at least 2.25 gram of said plant sterols per day. It will be appreciated that overconsumption of any product is inadvisable, therefore, preferably the edible composition is consumed in an amount of at most 100 gram, preferably at most 50 gram, more preferably at most 25 gram and most preferably at most 12 gram of said sterols per day.

Process

The edible composition according to the invention may suitably be made in a process comprising the step of preparing an aqueous dispersion comprising plant sterol particles and at least part of the water soluble emulsifier; and the step of combining said dispersion with fat powder comprising structuring fat.

It was found that the combination of at least part of a water soluble emulsifier according to the invention and plant sterol particles is required and that this combination of ingredients must be added as an aqueous dispersion. Failure to do so may result in an reduced effectiveness of a treatment, using said food composition, to lower blood cholesterol level. Furthermore, it may leads to unstable fat food compositions.

The aqueous dispersion can be prepared using well established methods as commonly used when preparing fat and water containing emulsions like for example edible fat continuous emulsions. For example a powder comprising plant sterol particles and water soluble emulsifier can simply be added to water under stirring. It is also possible to e.g. add the plant sterol particles and water soluble emulsifier as separate ingredients to water followed by stirring. If required the resulting dispersion may be processed by a microfluidizer to obtain the particle size distribution for the plant sterol particles according to the invention.

The invention is now illustrated by the following non limiting examples.

**Examples**

Particle size analysis

Particle size measurements of the aqueous dispersions were performed at 20 degrees Celsius using a static light scattering particle size analyzer (Mastersizer 2000, Malvern Instruments Ltd.).

The aqueous plant sterol dispersion was mixed well, four drops of the dispersion was added to two milliliters of water and mixed well. Subsequently a few drops of the obtained dilution were added to the approximately 130 ml of the measurement cell (Hydro 2000S) of the Mastersizer 2000 until the obscuration was within range. Next the measurement was started. No ultrasound was used before or during the measurement. For calculations the refractive index of sunflower oil (1.4694) and Mastersizer 2000 software version 5.54 was used. Particle sizes distributions are expressed as volume % below a set particle size in micrometers.

Determination of sterol particle distribution

Determination of the distribution of the sterol particles over the fat phase and aqueous phase was performed by Confocal Scattering Light Microscopy images. Sterol particles were labeled with a standard dye, such as Nile red or Nile blue. Any sterol particles in the fat were easily identifiable by their particular morphology as they appear as elongated agglomerates or platelet structures.

Preparation of aqueous dispersion comprising plant sterol particles

29.8 kg of Vegapure F90 ME (ex Cognis; containing 91.3% plant sterol and 7.7% skimmed milk powder (SMP)) was added to 85.2 kg of demineralised water at room temperature. The mixture was stirred in a pre-mix vessel using a blade stirrer. Subsequently the mixture was processed a microfluidizer (Microfluidics® M7125-20), equipped with auxiliary cell T60Z and down stream the interaction cell H10Z-8 and at a pressure of 1400 bar. After the interaction cell the dispersion was cooled to approximately 10 degrees Celsius using a heat exchanger. This was followed by 4 additional cycles at 1400 bar using both the auxiliary cell and the interaction cell to obtain sufficient particle size reduction. This resulted in a dispersion having a particle size distribution of 85 vol. % < 10 micrometer. The dispersion was stored at 5 degrees Celsius. The plant sterol dispersion was subsequently used to prepare Example 1.

Spreads with a composition according to table 1 were made using the process as described below.

Table 1, Spread composition (parts, w/w)

|  | Example 1 | Comparative A | Comparative B |
|---|---|---|---|
| FAT PHASE |  |  |  |
| Liquid oils | 29.55 | 24.40 | 31.22 |
| Fat powder | 4.3 |  |  |
| Hard stock |  | 5.3 | 3.5 |
| Dimodan HP | - | 0.2 | 0.2 |
| Lecithin | - | 0.1 | 0.05 |
| Colorant | 0.15 |  |  |
| Flavor | Trace | Trace | Trace |
| Colorant, vitamins, acidifier | Trace | Trace | Trace |
|  |  |  |  |
| AQUEOUS PHASE |  |  |  |
| Waxy maize starch | 2 | - | - |
| Tapioca starch | - | 2.5 | - |
| Gelatin |  | - | 1.25 |
| Plant sterol dispersion | 34.1 | - | - |
| Sterol esters |  | 12.5 | - |
| Potassium sorbate | 0.13 | 0.1 | 0.1 |
| Sunflower oil | 1 | - | - |
| Dimodan R-T/B | 0.15 | - | - |

(continued)

|  | Example 1 | Comparative A | Comparative B |
|---|---|---|---|
| Dimodan HP | 0.15 | - | - |
| Sweet whey powder | - | 0.4 | - |
| Tap water | Balance | Balance | Balance |

Dimodan® HP: molecularly distilled mono/diacylglyceride mixture derived from fully hardened palm oil (90% monoglyceride) ex Danisco DK.

Dimodan ® R-T/B: molecularly distilled mono/diacylglyceride mixture derived from hardened Rapeseed oil (90% monoglyceride) ex Danisco, DK.

Fat powder is a fat powder comprising structuring fat that was obtained using a supercritical melt micronisation process similar to the process described in 'Particle formation of ductile materials using the PGSS technology with supercritical carbon dioxide', P.Münüklü, Ph.D.Thesis, Delft University of Technology, 16-12-2005, Chapter 4, pp. 41-51; using an interesterified mixture of 65% dry fractionated palm oil stearin with an Iodine Value of 14 and 35% palm kernel oil.

Hard stock used for Comparative A was an interesterified mixture of 65% dry fractionated palm oil stearin with an Iodine Value of 14 and 35% palm kernel oil.

Hard stock used for Comparative B was an interesterified mixture of 60% palm kernel oil and 40% hydrogenated palm oil.

Preparation of Example 1

Preparation of fat phase

The fat powder was added to the liquid oil while stirring. The oil temperature was about 15 degrees Celsius. Vacuum was applied to remove all dissolved air from the resulting slurry. Subsequently colour and flavour were added to the slurry.

Preparation of the aqueous phase

The oil soluble emulsifiers were added to the sunflower oil while heating to dissolve the emulsifiers (as listed under aqueous phase in Table 1).The mix of sunflower and oil soluble emulsifier, together with starch, salt and potassium polysorbate, were added to the water under elevated temperature while stirring. After the resulting mixture cooled down to about 15 to 20 degrees Celsius the aqueous dispersion comprising the plant sterol particles as described above was added.

Preparation of emulsion

Both the fat phase and the aqueous phase were pumped to a mixing unit (pin-stirrer). The residence time in the pin-stirrer unit was between 10 and 110 seconds. The products so obtained at the exit of the pin-stirrer unit were stored at 5 degrees Celsius.

Preparation of Comparative examples A and B

All ingredients were stirred at 60 degrees Celsius in a premix tank. The premix was pumped into a standard votator line (configured of A and C units). The products so obtained at the exit of the votator line were stored at 5 degrees Celsius.

Analysis of spreads

In Example 1, more than 90 wt. % of the sterol particles, based on the total weight of the sterol particles were found to be present in the aqueous phase. In Comparative A and Comparative B no sterol particles in the aqueous phase could be observed.

Cholesterol Absorption Inhibition Clinical Study

The ability of the edible composition according to the invention to lower blood cholesterol level was evaluated in a clinical study. The study was a double-blind, cross-over, single dose study with 17 healthy, non-obese men (BMI 20-27 kg·m$^{-2}$, age range 20 - 45 yr). This was a randomized, double-blind, crossover study, with three treatment periods separated by a 4 week washout period. The cholesterol uptake inhibition of Example 1 was compared to Comparative spreads A and B. The spread of Example 1 contained 2.25 gram of free-sterols per 30 gram spread, Comparative A contained 2.25 grams of esterified sterols per 30 gram spread and Comparative B contained no added sterols. The inhibition of cholesterol absorption of Example 1 and Comparative A was calculated versus the comparative spread B, which did not contain added free sterols or esterified sterols. To quantify the level of cholesterol absorption 50 mg labeled D7-cholesterol was taken orally and 30 mg 3,4-$^{13}$C-cholesterol was dosed intravenously at the same time. The intravenous injection of 3,4-$^{13}$C-cholesterol serves as absolute reference with a known concentration of the labeled cholesterol in the blood-stream for a given dose.

Administration

Thirty grams of spread was added to a standardized meal based on an average Dutch breakfast. The composition of this standardized meal is shown in Table 2.

*Table 2 Composition of the standardized meal.*

| bread | three slices |
|---|---|
| cheese (48% fat) | 30 g |
| strawberry jam (45% fruit) | 15 g |
| test, reference or control product (as spread) | 30 g |
| water, juice | 100 ml |
| tea, coffee | 200 ml |

Participants were encouraged to minimize changes in composition of their habitual diet during the study periods. Volunteers were instructed to refrain from foods or food supplements enriched with phytosterols and also from any food supplement claiming to lower cholesterol.

Blood cholesterol level was measured via blood samples. After the first (t=0) blood sample an intravenous bolus of 30 mg of 3,4-$^{13}$C-cholesterol dissolved in 33 ml Liposyn III 10% was administered and participants ingested the 50mg of labeled D7 cholesterol as incorporated in the standardized breakfast, which also contains the study products (Example 1, Comparative A or Comparative B). Four hours after the infusion, when emptying of the stomach is completed, subjects were able to have lunch in the hospital and after that could return to their homes. Additional blood samples were collected at 48, 72, one at either 96, 120 or 144h, and finally at 168h after infusion (maximum of 13.5 ml at each time point).

Plasma labeled cholesterol concentrations (isotope enrichment) was determined with Gas chromatography-mass spectrometry and Isotope-ratio mass spectrometry.

After de-blinding the cholesterol absorption *inhibition* was calculated for each volunteer by taking the Comparative B treatment as 100% value and the actual value for the Example 1 or Comparative A treatment in relation to that. In formula:

$$inhibition(\%) = \frac{C - T}{C} \times 100\%$$

In which;

C = cholesterol absorption for Control (Comparative B), and,
T = cholesterol absorption for Treatment (Example 1 or Comparative A).

Results

As is shown in table 3 consumption of a spread comprising the edible composition according to the invention (Example 1) achieves a superior reduction in cholesterol absorption compared to well known alternative cholesterol lowering products (Comparative A).

Table 3 Cholesterol absorption results

|  | Average % absorption | % inhibition |
|---|---|---|
| Example 1 | 4.8 | 86.3 |
| Comparative A | 15.8 | 50.1 |
| Comparative B | 33.7 | - |

**Claims**

1. An edible composition comprising an aqueous phase, a fat phase and plant sterol particles for use in the prophylactic or curative treatment of elevated blood cholesterol level, wherein said edible composition comprises from 0.1 to 20 wt. % of plant sterol particles whereof at least 60 wt. %, based on the total weight of said plant sterol particles, is comprised by said aqueous phase, and wherein at least 70 vol. % of said plant sterol particles is smaller than 10 micrometer, and wherein said edible composition comprises a water soluble biopolymer based emulsifier with a molecular weight of at least 500, and wherein said edible composition is made in a process using fat powder comprising structuring fat, and wherein said treatment comprises the step of consuming said edible composition in an amount comprising at least 0.3 g of said plant sterols per thereof per day.

2. An edible composition according to claim 1, wherein the water soluble emulsifier is selected from the group consisting of proteins, glycoproteins, surface active polysaccharides and combinations thereof.

3. An edible composition according to claim 1 or claim 2, wherein the water soluble emulsifier is selected from the group consisting of full milk powder, skim milk powder, butter milk powder, sweet whey powder, whey protein, casein protein and combinations thereof.

4. An edible composition according to any one of claims 1 to 3 wherein at least 75 vol. %, preferably at least 80 vol. %, more preferably at least 85 vol. %, even more preferably at least 90 vol. % and still more preferably at least 95 vol. % of the plant sterol particles are smaller than 10 micrometer.

5. An edible composition according to any one of claim 1 to 4 wherein the amount of fat powder used is from 1 to 20 wt. %, preferably from 2 to 15 wt. %, more preferably from 4 to 12 wt. %, even more preferably from 6 to 8 wt. % and still more preferably from 3 to 5 wt. %.

6. An edible composition according to any one of claims 1 to 5 wherein the amount of plant sterol particles is from 2 to 15 wt. %, preferably from 4 to 10 wt. % and more preferably from 6 to 8 wt.%.

7. An edible composition according to any one of claims 1 to 6, wherein the amount of water soluble emulsifier is from 0.01 to 5wt., preferably from 0.1 to 2wt. and more preferably form 0.1 to 0.5 wt.

8. An edible composition according to any one of claims 1 to 7, wherein the weight ratio of water soluble emulsifier to plant sterol particles is from 10:1 to 1:80, preferably 8:1 to 1:40, more preferably 6:1 to 1:20, even more preferably 4:1 to 1:15 and still more preferably 2:1 to 1:10.

9. An edible composition according to any one of claims 1 to 8, wherein at least 70 wt. %, more preferably at least 85 wt. % and most preferably at least 90 wt. % of said sterols, based of the total weight of said sterol particles, is comprised by said aqueous phase.

10. An edible composition according to any one of claims 1 to 9, wherein said composition is a spread, dressing, mayonnaise, shortening, filling, topping, cream, soup, beverage or sauce.

11. An edible composition according to any one of claims 1 to 10 for use in said treatment, wherein said edible composition is consumed as a meal or as part of a meal, preferably wherein said meal comprises from 2 to 50 grams of fat, more preferably from 3 to 10 grams of fat and most preferably from 4 to 6 grams of fat.

12. An edible composition according to any one of claims 1 to 11 for use in said treatment, wherein at least 0.5 gram, preferably at least 1 gram, more preferably at least 2 gram and most preferably at least 2.25 gram and at most 100 gram, preferably at most 50 gram, more preferably at most 25 gram and most preferably at most 12 gram of said sterols is consumed per day.

13. An edible composition according to any one of claims 1 to 12, for use in said treatment, wherein the subject is a human, preferably a human with elevated blood cholesterol level, more preferably a human with hypercholesterolemia.

14. An edible composition according to any one of claims 1 to 13, wherein said process in addition comprises the step of preparing an aqueous dispersion comprising plant sterol particles and at least part of the water soluble emulsifier, and the step of combining said dispersion with fat powder comprising structuring fat.

15. An edible composition according to any one of claims 1 to 14, wherein said fat powder comprises structuring fat obtainable by supercritical melt micronisation.


**Patentansprüche**

1. Essbare Zusammensetzung, umfassend eine wässrige Phase, eine Fettphase und Pflanzensterolpartikel zur Verwendung bei der prophylaktischen oder heilenden Behandlung erhöhten Blutcholesterinspiegels, wobei die essbare Zusammensetzung 0,1 bis 20 Gew.-% der Pflanzensterolpartikel umfasst, wovon mindestens 60 Gew.-%, bezogen auf das gesamte Gewicht der Pflanzensterolpartikel, von der wässrigen Phase umfasst sind und worin mindestens 70 Vol.-% der Pflanzensterolpartikel kleiner als 10 Mikrometer sind und wobei die essbare Zusammensetzung einen auf einem wasserlöslichen Biopolymer-basierenden Emulgator mit einem Molekulargewicht von mindestens 500 umfasst und wobei die essbare Zusammensetzung anhand eines Verfahrens hergestellt wird, bei dem ein Fettpulver verwendet wird, das Strukturierungsfett umfasst, und wobei die Behandlung den Schritt des Verzehrs der essbaren Zusammensetzung in einer Menge umfasst, die mindestens 0,3 g der Pflanzensterole pro Tag umfasst.

2. Essbare Zusammensetzung nach Anspruch 1, worin der in Wasser lösliche Emulgator aus der aus Proteinen, Glycoproteinen, oberflächenaktiven Polysacchariden und Kombinationen davon bestehenden Gruppe ausgewählt ist.

3. Essbare Zusammensetzung nach Anspruch 1 oder Anspruch 2, worin der wasserlösliche Emulgator aus der aus Vollmilchpulver, Magermilchpulver, Buttermilchpulver, Süßmolkenpulver, Molkenpulver, Caseinprotein und Kombinationen davon bestehenden Gruppe ausgewählt ist.

4. Essbare Zusammensetzung nach irgendeinem der Ansprüche 1 bis 3, worin mindestens 75 Vol.-%, vorzugsweise mindestens 80 Vol.-%, bevorzugter mindestens 85 Vol.-%, sogar bevorzugter mindestens 90 Vol.-% und noch bevorzugter mindestens 95 Vol.-% der Pflanzensterolpartikel kleiner als 10 Mikrometer sind.

5. Essbare Zusammensetzung nach irgendeinem der Ansprüche 1 bis 4, worin die Menge des verwendeten Fettpulvers 1 bis 20 Gew.-%, vorzugsweise 2 bis 15 Gew.-%, bevorzugter 4 bis 12 Gew.-%, sogar bevorzugter 6 bis 8 Gew.-% und noch bevorzugter 3 bis 5 Gew.-% beträgt.

6. Essbare Zusammensetzung nach irgendeinem der Ansprüche 1 bis 5, worin die Menge der Pflanzensterolpartikel 2 bis 15 Gew.-%, vorzugsweise 4 bis 10 Gew.-% und bevorzugter 6 bis 8 Gew.-% beträgt.

7. Essbare Zusammensetzung nach irgendeinem der Ansprüche 1 bis 6, worin die Menge des wasserlöslichen Emulgators 0,01 bis 5 Gew.-%, vorzugsweise 0,1 bis 2 Gew.-% und bevorzugter 0,1 bis 0,5 Gew.-% beträgt.

8. Essbare Zusammensetzung nach irgendeinem der Ansprüche 1 bis 7, worin das Gewichtsverhältnis des wasserlöslichen Emulgators zu den Pflanzensterolpartikeln 10:1 bis 1:80, vorzugsweise 8:1 bis 1:40, bevorzugter 6:1 bis 1:20, sogar bevorzugter 4:1 bis 1:15 und noch bevorzugter 2:1 bis 1:10 beträgt.

9. Essbare Zusammensetzung nach irgendeinem der Ansprüche 1 bis 8, worin mindestens 70 Gew.-%, vorzugsweise mindestens 85 Gew.-% und höchst bevorzugt mindestens 90 Gew.-% der Sterole, bezogen auf das gesamte Gewicht der Sterolpartikel, von der wässrigen Phase umfasst sind.

**10.** Essbare Zusammensetzung nach irgendeinem der Ansprüche 1 bis 9, worin die Zusammensetzung ein Aufstrich, ein Dressing, Mayonnaise, ein Backfett, eine Füllung, eine Garnierung, eine Creme, eine Suppe, ein Getränk oder eine Soße ist.

**11.** Essbare Zusammensetzung nach irgendeinem der Ansprüche 1 bis 10 zur Verwendung bei der besagten Behandlung, worin die essbare Zusammensetzung als Mahlzeit oder als Teil einer Mahlzeit verzehrt wird, worin die Mahlzeit vorzugsweise 2 bis 50 Gramm Fett, bevorzugter 3 bis 10 Gramm Fett und höchst bevorzugt 4 bis 6 Gramm Fett umfasst.

**12.** Essbare Zusammensetzung nach irgendeinem der Ansprüche 1 bis 11 zur Verwendung bei der besagten Behandlung, worin mindestens 0,5 Gramm, vorzugsweise mindestens 1 Gramm, bevorzugter mindestens 2 Gramm und höchst bevorzugt mindestens 2,25 Gramm und höchstens 100 Gramm, vorzugsweise höchstens 50 Gramm, bevorzugter höchstens 25 Gramm und höchst bevorzugt höchstens 12 Gramm der Sterole pro Tag verzehrt werden.

**13.** Essbare Zusammensetzung nach irgendeinem der Ansprüche 1 bis 12 zur Verwendung bei der besagten Behandlung, worin das Subjekt ein Mensch ist, vorzugsweise ein Mensch mit angehobenem Blutcholesterinspiegel, bevorzugter ein Mensch mit Hypercholesterinämie.

**14.** Essbare Zusammensetzung nach irgendeinem der Ansprüche 1 bis 13, wobei das Verfahren der Zugabe den Schritt der Herstellung einer wässrigen Dispersion umfasst, umfassend Pflanzensterolpartikel und mindestens einen Teil des wasserlöslichen Emulgators und umfassend den Schritt des Kombinierens der Dispersion mit einem Strukturierungsfett enthaltenden Fettpulver.

**15.** Essbare Zusammensetzung nach irgendeinem der Ansprüche 1 bis 14, worin das Fettpulver Strukturierungsfett, erhältlich durch superkritische Schmelzmikronisierung, umfasst.

**Revendications**

**1.** Composition comestible comprenant une phase aqueuse, une phase de graisse et des particules de stérol végétal pour une utilisation dans le traitement prophylactique ou curatif d'une teneur élevée en cholestérol dans le sang, dans laquelle ladite composition comestible comprend de 0,1 à 20 % en masse de particules de stérol végétal dont au moins 60 % en masse, rapportés à la masse totale desdites particules de stérol végétal, sont comprises par ladite phase aqueuse, et dans laquelle au moins 70 % en volume desdites particules de stérol végétal sont inférieurs à 10 micromètres, et dans laquelle ladite composition comestible comprend un émulsionnant à base de biopolymère soluble dans l'eau avec une masse moléculaire d'au moins 500, et dans laquelle ladite composition comestible est fabriquée dans un procédé utilisant de la poudre de graisse comprenant une graisse structurante, et dans laquelle ledit traitement comprend l'étape de consommation de ladite composition comestible dans une quantité comprenant au moins 0,3 g desdits stérols végétaux pour celle-ci par jour.

**2.** Composition comestible selon la revendication 1, dans laquelle ledit émulsionnant soluble dans l'eau est choisi dans le groupe constitué de protéines, de glycoprotéines, de polysaccharides actifs en surface et de combinaisons de ceux-ci.

**3.** Composition comestible selon la revendication 1 ou la revendication 2, dans laquelle l'émulsionnant soluble dans l'eau est choisi dans le groupe constitué de poudre de lait entier, de poudre de lait écrémé, de poudre de babeurre, de poudre de lactosérum doux, de protéine de lactosérum, de protéine de caséine et de combinaisons de celles-ci.

**4.** Composition comestible selon l'une quelconque des revendications 1 à 3, dans laquelle au moins 75 % en volume, de préférence au moins 80 % en volume, encore mieux au moins 85 % en volume, bien mieux encore au moins 90 % en volume et particulièrement de préférence au moins 95 % en volume des particules de stérol végétal sont inférieurs à 10 micromètres.

**5.** Composition comestible selon l'une quelconque des revendications 1 à 4, dans laquelle la quantité de poudre de graisse utilisée est de 1 à 20 % en masse, de préférence de 2 à 15 % en masse, encore mieux de 4 à 12 % en masse, bien mieux encore de 6 à 8 % en masse et particulièrement de préférence de 3 à 5 % en masse.

**6.** Composition comestible selon l'une quelconque des revendications 1 à 5, dans laquelle la quantité de particules de stérol végétal est de 2 à 15 % en masse, de préférence de 4 à 10 % en masse et encore mieux de 6 à 8 % en masse.

7. Composition comestible selon l'une quelconque des revendications 1 à 6, dans laquelle la quantité d'émulsionnant soluble dans l'eau est de 0,01 à 5 % en masse, de préférence de 0,1 à 2 % en masse et encore mieux de 0,1 à 0,5 % en masse.

8. Composition comestible selon l'une quelconque des revendications 1 à 7, dans laquelle le rapport massique d'émulsionnant soluble dans l'eau aux particules de stérol végétal est de 10:1 à 1:80, de préférence de 8:1 à 1:40, encore mieux de 6:1 à 1:20, bien mieux encore de 4:1 à 1:15 et particulièrement de préférence de 2:1 à 1:10.

9. Composition comestible selon l'une quelconque des revendications 1 à 8, dans laquelle au moins 70 % en masse, encore mieux au moins 85 % en masse et bien mieux encore au moins 90 % en masse desdits stérols, rapportés à la masse totale desdites particules de stérol, sont compris par ladite phase aqueuse.

10. Composition comestible selon l'une quelconque des revendications 1 à 9, dans laquelle ladite composition est une pâte à tartiner, un assaisonnement, une mayonnaise, une matière grasse, une farce, une crème fouettée, une crème, une soupe, une boisson ou une sauce.

11. Composition comestible selon l'une quelconque des revendications 1 à 10 pour une utilisation dans ledit traitement, dans laquelle ladite composition comestible est consommée comme un repas ou comme une partie d'un repas, de préférence dans laquelle ledit repas comprend de 2 à 50 grammes de graisse, encore mieux de 3 à 10 grammes de graisse et bien mieux encore de 4 à 6 grammes de graisse.

12. Composition comestible selon l'une quelconque des revendications 1 à 11 pour une utilisation dans ledit traitement, dans laquelle au moins 0,5 gramme, de préférence au moins 1 gramme, encore mieux au moins 2 grammes et bien mieux encore au moins 2,25 grammes et au plus 100 grammes, de préférence au plus 50 grammes, encore mieux au plus 25 grammes et bien mieux encore au plus 12 grammes desdits stérols sont consommés par jour.

13. Composition comestible selon l'une quelconque des revendications 1 à 12, pour une utilisation dans ledit traitement, dans laquelle le sujet est un être humain, de préférence un être humain avec une teneur élevée en cholestérol dans le sang, encore mieux un être humain avec une hypercholestérolémie.

14. Composition comestible selon l'une quelconque des revendications 1 à 13, dans laquelle ledit procédé dans l'addition comprend l'étape de préparation d'une dispersion aqueuse comprenant des particules de stérol végétal et au moins une partie de l'émulsionnant soluble dans l'eau ; et l'étape de combinaison de ladite dispersion avec une poudre de graisse comprenant une graisse structurante.

15. Composition comestible selon l'une quelconque des revendications 1 à 14, dans laquelle ladite poudre de graisse comprend une graisse structurante pouvant être obtenue par micronisation à l'état de fusion supercritique.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0228204 A
- WO 9858554 A
- US 20060035871 A1
- WO 0021490 A
- WO 2011144405 A
- EP 1142494 A
- EP 1839501 A
- WO 2008125380 A

**Non-patent literature cited in the description**

- **MATTSON.** *Am J Clin Nutr,* 1982
- **POUTEAU.** *Eur J Nutr,* 2003
- **VANSTONE.** *Am J Clin Nutr,* 2002
- **RICHELLE.** *Am J Clin Nutr,* 2004
- **SHIN.** *Ann Nutr & Met,* 2004
- **SUDHOP.** *Drug Res.,* 2003
- **S.P. KOCHHAR.** Influence of Processing on Sterols of Edible Vegetable Oils. *Prog. Lipid Res.,* vol. 22, 161-188
- **P.MÜNÜKLÜ, PH.D.THESIS.** Delft University of Technology. 16 December 2005, 41-51